# EUROPEAN PATENT APPLICATION

(11) **EP 2 471 384 A1**
(43) Date of publication of application: **04.07.2012**
(21) Application number: 10197294.1
(22) Date of filing: 29.12.2010
(51) Int. Cl.: A23L 1/30, A61K 31/23, A61K 9/00

(54) **Suspension formulations of cetyl myristate and/or cetyl palmitate**

(71) Applicant: Deva Holding Anonim Sirketi, Kucukcekmece/Istanbul (TR)
(72) Inventor: Haas, Philipp Daniel, 34303, ISTANBUL (TR); Firat, Omer Faruk, 34303, ISTANBUL (TR); Kademit, Levent, 34303, ISTANBUL (TR); Koc, Fikret, 34303, ISTANBUL (TR); Sivasligil, Ramazan, 34303, ISTANBUL (TR)

(57) **Abstract**

This invention is related to pharmaceutical or dietary supplement suspension formulations of cetylated fatty acids, especially cetyl myristate or cetyl palmitate or combination of cetyl myristate and cetyl palmitate.

## Description

### Technical Field

This invention is related to pharmaceutical and/or dietary supplement suspension formulations of cetylated fatty acids, especially cetyl myristate or cetyl palmitate or combination of cetyl myristate and cetyl palmitate.

### Background Art

Cetyl palmitate is derived from the fatty acid, palmitic acid which occurs as the glycerol ester in many oils and fats such as palm oil or Chinese vegetable tallow. A synthetic method of preparation is to react palmitoyl chloride and cetyl alcohol in the presence of magnesium. See the Merck Index, 12th edition at page 336. Reference is also made to US US3169099 A (SOCONY MOBIL OIL CO INC) 02.09.1965 patent which discloses a biosynthetic method of producing cetyl palmitate.

US 4,113,881 A (DIEHL HARRY WELDON) 12.09.1978 discloses that the administration of an effective amount of cetyl myristoleate to a mammal is useful in inhibiting or relieving the symptoms of inflammatory rheumatoid arthritis in mammals.

US 5569676 A (DIEHL, HARRY W) 29.10.1996 US 5,569,676 discloses the use of cetyl myristoleate in the treatment of osteo-arthritis.

WO 01/85162 A (MERACOL CORP LTD ET.AL.) 15.11.2001 discloses the use of cetyl myristate and/or cetyl palmitate in the treatment of irritable bowel syndrome or disease. Patent embraces that cetyl myristate comprises 50-98 wt.% of the mixture, preferably, the myristate and palmitate are in a weight ratio of 95:5. The oral dosage unit is a capsule and contains 5-400 mg of the cetyl myristate or the mixture of the cetyl myristate and the cetyl palmitate. It also includes an excipient and/or diluent, preferably silicon dioxide, calcium phosphate and/or magnesium oxide. Preferably said mixture is in a capsule and it includes a pharmaceutically acceptable excipient and/or diluents. Preferably the dosage unit includes silicon dioxide, calcium phosphate and/or magnesium oxide. Liquid formulation, where an amount of liquid equivalent to at least 4 capsules is prescribed which is to be taken 3 times daily. That is 4200 mg of cetyl myristate or the mixture of cetyl myristate and cetyl palmitate.That mixture comprises by weight 95% cetyl myristate and 5% cetyl palmitate by weight In addition added excipients were present in the non gelatin two part capsule case.

WO 01/85163 A (MERACOL CORP LTD ET.AL.) 15.11.2001 discloses the use of cetyl myristate and/or cetyl palmitate in the treatment of eczema and/or psoriasis. Accordingly, capsule also includes a pharmaceutically acceptable excipient and/or diluent. These are silicon dioxide, calcium phosphate and/or magnesium oxide. The dosage unit can also be a wax-like solid or can be an orally consumable liquid composition (eg; made up with a general pharmacy type carrier such as methyl cellulose).

WO 2005/118070 A (MERACOL CORP LTD ET.AL.) 15.12.2005 discloses the treatment of multiple sclerosis with the use of cetyl myristate and/or cetyl palmitate.The cetyl myristate; or combination of cetyl myristate and cetyl palmitate is administered simultaneously, separately or sequentially.

WO 03/018731 A (MERACOL CORP LTD) 06.03.2003 defines the process prepares a mixture of cetyl myristate (50-98 wt.%) and cetyl palmitate, for use in the formulation of cosmetics and pharmaceuticals.

WO 03/045374 A (MERACOL CORP LTD ET.AL.) 05.06.2003 discloses the use of cetyl myristate and/or cetyl palmitate in a method of treatment and/or prophylaxis of a mammal for at least the symptoms of treating asthma, chronic obstructive pulmonary disease and/or other respiratory difficulties.

WO 03/026640 A (MERACOL CORP LTD ET.AL.) 03.04.2003 discloses the use of cetyl myristate and/or cetyl palmitate in the treatment of food allergies and/or food intolerances.

WO 01/85164 A (MERACOL CORP LTD ET.AL.) 15.11.2001 discloses the use of cetyl myristate and/or cetyl palmitate in the treatment of herpes.

### Summary of invention

This invention discloses eligible suspension formulations of cetylated fatty acids, especially cetyl myristate or cetyl palmitate or combination of cetyl palmitate and cetyl myristate.

### Technical Problem

Cetyl myristate or cetyl palmitate or combination of cetyl myristate and cetyl palmitate can be used as active pharmaceutical ingredients (API) in pharmaceutical or dietary supplement formulations in addition to their excipient properties. However suspending of those is very hard, since particles of cetyl myristate and cetyl palmitate are immediately sedimendated and resuspending becomes problematic or impossible. Suspension needs adequate shaking of the container to resuspend the drug uniformly prior to dosing. Redispersion difficulty of the drug from a sediment will result in under- and overdosing. Therefore suspension should maintain its content uniformity for a period of time at least patients can be properly ingest it.

### Solution to Problem

It is invented that in formulations, when certain excipients are used in specific ranges, agglomeration is not occurred and/or resuspending is improved. In the terms of solution, rapid sedimentation is reduced by increasing the viscosity of the liquid. Departing from this point of view, rapid sedimentation of a solution is reduced as the viscosity of the liquid is increased. The problem is eliminated via use of viscosity increasing agent in a specific concentration.

### Description of embodiments

Rapid sedimentation can be reduced by increasing the viscosity of the liquid phase. It is achieved by using of viscosity increasing agent in a specific amount per mL. Thus viscosity enhancing agent is used from about 0.1 mg/mL to about 50 mg/mL. Preferably, viscosity enhancing agent is from about 0.1 mg/mL to about 25 mg/mL. More preferably, viscosity enhancing agent is from about 1 mg/mL to about 15 mg/mL. Most preferably viscosity enhancing agent is about 5 mg/mL.

Viscosity enhancing agent is, but not limited to xantham gum, guar gum, acacia, alginic acid, sodium alginate, propylene glycol alginate, povidone, carbomer, salts of carboxymethylcellulose, methylcellulose, ethylcellulose, hydroxyethyl cellulose, hydroxypropyl cellulose, hydroxypropyl methylcellulose, bentonite, polydextrose, carrageenan, sucrose, sorbitol, xylitol, dextrose, fructose, malitol, gelatin, tragacanth, a polyvinyl alcohol, cetearyl alcohol, colloidal silicon dioxide, mixtures thereof and the like.

In accordance with this invention cetyl myristate is used from about 10 mg/mL to about 200 mg/mL. Preferably, cetyl myristate is from about 30 mg/mL to about 100 mg/mL. More preferably, cetyl myristate is from about 50 mg/mL to about 80 mg/mL. Most preferably cetyl myristate is about 66,66 mg/mL.

In another aspect of this invention, cetyl palmitate is used from about 0.1 mg/mL to about 30 mg/mL. Preferably, cetyl palmitate is from about 1 mg/mL to about 10 mg/mL. More preferably, cetyl palmitate is from about 2 mg/mL to about 5 mg/mL. Most preferably cetyl palmitate is about 3.34 mg/mL.

In accordance with this invention preservative is used from about 0.1 mg/mL to about 30 mg/mL. Preferably, preservative is from about 0.2 mg/mL to about 20 mg/mL. More preferably, preservative is from about 0.3 mg/mL to about 6 mg/mL. Most preferably preservative is about 3 mg/mL.

Preservative is, but not limited to, sodium benzoate, benzoic acid, ethylenediaminetetraacetic acid, sorbic acid, benzethonium chloride, benzalkonium chloride, bronopol, butyl paraben, methyl paraben, ethylparaben, propyl paraben, thiomerosol, sodium propionate, chlorhexidine, chlorobutanol, chlorocresol, cresol, imidurea, phenol, phenylmercuric salts, potassium sorbate, propylene glycol, mixtures thereof and the like.

In accordance with this invention sweetener is also used. Accordingly sweetener is used from about 10 mg/mL to about 500 mg/mL. Preferably, sweetener is from about 30 mg/mL to about 200 mg/mL. More preferably, sweetener is from about 110 mg/mL to about 180 mg/mL. Most preferably sweetener is about 150 mg/mL.

Sweeteners are but not limited to, corn syrup, dextrose, invert sugar, fructose, saccharin, aspartame, acesulfame-K, Stevia rebaudiana, sucralose, sorbitol, mannitol, xylitol, mixtures thereof and the like.

In accordance with this invention flavor is also used. Accordingly flavour is used from about 0.01 mg/mL to about 3 mg/mL. Preferably, flavor is from about 0.02 mg/mL to about 1 mg/mL. More preferably, flavor is from about 0.04 mg/mL to about 0.5 mg/mL. Most preferably flavor is about 0.08 mg/mL.

Flavors are, but not limited to, cinnamon oil,essence of apple, essence of pear, essence of peach, essence of grape, essence of strawberry, essence of raspberry, essence of cherry, essence of plum, essence of pineapple, essence of apricot, oil of wintergreen, peppermint oils, clove oil, bay oil, anise oil, eucalyptus, thyme oil, cedar leaf oil, oil of nutmeg, oil of sage, oil of bitter almonds, cassia oil, citrus oils such as lemon, orange, grape, lime and grapefruit, vanilla, benzaldehyde, aldehyde C-8, aldehyde C-9, aldehyde C-12, tolyl aldehyde, mixtures thereof and the like.

According to this invention flavour enhancer is also used.Accordingly flavour enhancer is used from about 0.1 mg/mL to about 10 mg/mL. Preferably, flavour enhancer is from about 0.5 mg/mL to about 5 mg/mL. More preferably,flavour enhancer is from about 0.8 mg/mL to about 3 mg/mL. Most preferably flavour enhancer is about 1 mg/mL.

Flavor enhancer is but not limited to citric acid monohydrate, ethyl maltol, maltol, monosodium glutamate,neohesperidin dihydrochalcone,neotame,thaumatin,trehalose, disodium guanylate, disodium inosinate, mixtures thereof and the like.

According to this invention antifoaming agent is also used. Antifoaming agent is used from about 0.01 mg/mL to about 5 mg/mL. Preferably, antifoaming agent is from about 0.2 mg/mL to about 3 mg/mL. More preferably, antifoaming agent is from about 0.4 mg/mL to about 1 mg/mL. Most preferably antifoaming agent is about 0.5 mg/mL.

Anti-foaming agent is selected from the group consisting of silicon oil, simethicone, dimethicone, oleyl alcohol, polypropylene glycol, mixtures thereof and the like.

In another aspect, pH adjuster is used from about 0.01 mg/mL to about 10 mg/mL. Preferably, pH adjuster is from about 1 mg/mL to about 8 mg/mL. More preferably, pH adjuster is from about 1.5 mg/mL to about 6 mg/mL. Most preferably pH adjuster is about 2.1 mg/mL.

pH adjuster is but not limited to hydrochloric acid, sodium hydroxide, citric acid, phosphoric acid, lactic acid, tartaric acid, succinic acid, mixtures thereof and the like.

Deionized water is used as solvent in this invention.

### Example

**Table 1**

| **Function** | **mg/mL** |
|---|---|
| Cetyl Myristate(Active Pharmaceutical Ingredient) | 333.30 mg / 5 mL |
| Cetyl Palmitate(Active Pharmaceutical Ingredient) | 16.70 mg / 5 mL |
| Preservative | 15.00 mg / 5 mL |
| Flavor Enhancer | 5.00 mg/5mL |
| Sweetener | 750.00 mg / 5 mL |
| Antifoaming agent | 2.50mg/5mL |
| Viscosity Enhancer | 25.00 mg / 5 mL |
| Flavor | 0.4mg/5mL |
| pH adjuster | 10.50 mg / 5 mL |

## Claims

1. A pharmaceutical or dietary supplement suspension formulation of combination of cetyl myristate and cetyl palmitate comprising cetyl myristate is from 10 mg/mL to 200 mg/mL, cetyl palmitate is from 0.1 mg/mL to 30 mg/mL, viscosity enhancing agent is from 0.1 mg/mL to 50 mg/mL, preservative is from 0.1 mg/mL to 30 mg/mL, sweetener is from 10 mg/mL to 500 mg/mL, flavour is from 0.01 mg/mL to 3 mg/mL, flavour enhancer agent is from 0.1 mg/mL to 10 mg/mL, antifoaming is from 0.01 mg/mL to 5 mg/mL, pH adjuster is from 0.01 mg/mL to 10 mg/mL.

2. As claimed in claim 1, suspension formulation preferably comprising viscosity enhancing agent is from 0.1 mg/mL to 25 mg/mL, cetyl myristate is from 30 mg/mL to 100 mg/mL, cetyl palmitate is from 1 mg/mL to 10 mg/mL, preservative is from 0.2 mg/mL to 20 mg/mL, sweetener is from 30 mg/mL to 200 mg/mL, flavor is from 0.02 mg/mL to 1 mg/mL, flavour enhancer agent is from 0.5 mg/mL to 5 mg/mL, antifoaming agent is from 0.2 mg/mL to 3 mg/mL, pH adjuster is from 1 mg/mL to 8 mg/mL.

3. As claimed in claim 2, suspension formulation more preferably comprising viscosity enhancing agent is from 1 mg/mL to 15 mg/mL, cetyl myristate is from 50 mg/mL to 80 mg/mL, cetyl palmitate is from 2 mg/mL to 5 mg/mL, preservative is from 0.3 mg/mL to 6 mg/mL, sweetener is from 110 mg/mL to 180 mg/mL, flavor is from 0.04 mg/mL to 0.5 mg/mL, flavour enhancer agent is from 0.8 mg/mL to 3 mg/mL, antifoaming is from 0.4 mg/mL to 1 mg/mL, pH adjuster is from about 1,5 mg/mL to about 6 mg/mL.

4. As claimed in claim 3, suspension formulation most preferably comprising viscosity enhancing agent is 5 mg/mL, cetyl myristate is 66,66 mg/mL, cetyl palmitate is 3,34 mg/mL, preservative is 3 mg/mL, sweetener is 150 mg/mL, flavor is 0.08 mg/mL, flavour enhancer is 1mg/mL, antifoaming agent is 0.5 mg/mL,pH adjuster is 2.1 mg/mL.
